# EUROPEAN PATENT SPECIFICATION

(11) **EP 1 867 713 B1**
(45) Date of publication and mention of the grant of the patent: **24.08.2011**
(21) Application number: 06730571.4
(22) Date of filing: 30.03.2006
(51) Int. Cl.: C12N 1/04, C12N 1/14, C12N 1/16, C12N 1/20

(54) **METHOD OF IMPROVING STORAGE STABILITY OF DRIED MICROORGANISMS**
VERFAHREN ZUR VERBESSERUNG DER LAGERSTABILITÄT GETROCKNETER MIKROORGANISMEN
MÉTHODE D'AMÉLIORATION DE LA STABILITÉ AU STOCKAGE D'UN MICROORGANISME SÉCHÉ

(30) Priority: 30.03.2005 JP 2005098211
(43) Date of publication of application: 19.12.2007
(73) Proprietor: Kyowa Hakko Bio Co., Ltd, Chiyoda-ku Tokyo 100-8185 (JP)
(72) Inventor: KAMIYA, Toshikazu c/o Healthcare Products Development Center, Tsukuba-shi, Ibaraki 305-0841 (JP); KIMURA, Masao c/o Healthcare Products Development Center, Tsukuba-shi, Ibaraki 305-0841 (JP); SAKAI, Yasushi c/o Healthcare Products Development Center, Tsukuba-shi, Ibaraki 305-0841 (JP)
(74) Representative: Harding, Charles Thomas
(86) International application number: PCT/JP2006/306623
(87) International publication number: WO 2006/106806

(56) References cited:
- EP-A1- 1 867 330
- WO-A1-91/11509
- WO-A2-96/28008
- JP-A- 05 292 943
- JP-A- 08 205 857
- JP-A- 54 076 886
- JP-A- 60 172 280
- JP-A- 2003 219 862

## Description

### Technical Field

The present invention relates to a method of improving storage stability of dried microorganisms, compositions containing dried microorganisms and a process for producing the compositions, and a method of preserving dried microorganisms.

### Background Art

Some microorganisms, such as lactic acid bacteria, are known as useful intestinal microorganisms, and products containing such microorganisms are commercially available, including yoghurts and health foods.

While viable microorganisms are used for some products, such as yoghurts, microorganisms are often used in dried form, that is, as dried microorganisms, for preparations such as tablets.

However, the viability of dried microorganisms is lowered during storage before or after preparation.

A variety of modifications have been attempted to solve this problem, that is, to improve storage stability of dried microorganisms.

One of typical methods is the addition of silica gel or skim milk. Another known method is the addition of arginine, ornithine, serine, or a salt thereof before freeze-drying of microorganisms (see Patent Document 1).

However, a method capable of further improving storage stability of dried microorganisms has been demanded.
Patent Document 1: Japanese Unexamined Patent Application Publication No. 2003-219862

### Disclosure of Invention

### Problems to be Solved by the Invention

An object of the present invention is to provide a method of improving storage stability of dried microorganisms, a composition containing dried microorganisms and a process for producing the composition, or a process for storing dried microorganisms.

### Means for Solving the Problems

The present invention relates to Items (1) to (6) below.
(1) A method of improving storage stability of dried microorganisms, which comprises allowing the dried microorganisms to coexist with an L-arginine acidic amino acid salt.
(2) A process for producing a composition containing dried microorganisms, which comprises a step of mixing the dried microorganisms with an L-arginine acidic amino acid salt.
(3) The process according to Item (2), wherein the composition is a preparation.
(4) A composition containing dried microorganisms and an L-arginine acidic amino acid salt.
(5) A preparation containing dried microorganisms and an L-arginine acidic amino acid salt.
(6) A method of storing dried microorganisms, which comprises storing the dried microorganisms in the presence of an L-arginine acidic amino acid salt.

### Effect of the Invention

The present invention can provide a method of improving storage stability of dried microorganisms, a composition containing dried microorganisms with improved storage stability and a process for producing the same, or a method for preserving dried microorganisms.

### Best Mode for Carrying Out the Invention

The microorganisms used in the present invention may be any type of microorganisms, such as bacteria or yeast, that can be preserved by drying. Examples of the microorganisms include microorganisms belonging to the genus Bifidobacterium, such as Bifidobacterium longum, Bifidobacterium adolescentis, Bifidobacterium infantis, Bifidobacterium bifidum, and Bifidobacterium breve; microorganisms belonging to the genus Lactobacillus, such as Lactobacillus casei, Lactobacillus acidophilus, and Lactobacillus plantarum; microorganisms belonging to the genus Streptococcus, such as Streptococcus faecalis; microorganisms belonging to the genus Escherichia, such as Escherichia coli; microorganisms belonging to the genus Corynebacterium, such as Corynebacterium glutamicum and Corynebacterium ammoniagenes; microorganisms belonging to the genus Bacillus, such as Bacillus subtilis; and microorganisms belonging to the genus Saccharomyces, such as Saccharomyces cerevisiae. Preferably, microorganisms belonging to the genera Bifidobacterium, Lactobacillus, Streptococcus, and Saccharomyces are used. More preferably, microorganisms generally classified as lactic acid bacteria are used, including the genera Bifidobacterium, Lactobacillus, and Streptococcus.

Among lactic acid bacteria, Bifidobacterium longum, Bifidobacterium adolescentis, Bifidobacterium infantis, Bifidobacterium bifidum, Bifidobacterium breve, Lactobacillus casei, Lactobacillus acidophilus, Lactobacillus plantarum, and Streptococcus faecalis, for example, are preferably used.

Such microorganisms are cultivated according to a general method for cultivating microorganisms to prepare a culture. The method for cultivation can be either solid cultivation using a solid culture medium or liquid cultivation using a liquid culture medium.

For cultivation using a solid culture medium, microorganisms grown on the medium can be used as a culture directly together with the medium or after the microorganisms are collected from the medium by a method such as scraping.

For cultivation using a liquid culture medium, the culture solution can be directly used as a culture, or the microorganisms can be removed from the culture solution by a method such as filtration or centrifugal separation before being used as a culture.

The culture thus prepared can be dried by a method such as freeze-drying using a dryer or spray drying using a spray dryer to prepare dried microorganisms.

Alternatively, commercially available dried microorganisms can be used.

The L-arginine acidic amino acid salt used in the present invention may be, for example, a natural product, a product obtained using microorganisms or a material derived therefrom, or a product obtained by chemical synthesis, and the product used can be either a purified product or a crude product. Also, a commercially available product can be used.

As acidic amino acid salt of L-arginine acidic amino acid salt, glutamate salt or aspartate can be mentioned.

A minimum water content is preferred for the L-arginine acidic amino acid salt. The water content is preferably 3% by weight or less, more preferably 1% by weight or less, still more preferably 0.3% by weight or less.

The storage stability of the dried microorganisms can be improved by allowing them to coexist with the L-arginine acidic amino acid salt.

The dried microorganisms may be allowed to coexist with the L-arginine acidic amino acid salt by any method. A preferred example is the mixing of the dried microorganisms with the L-arginine acidic amino acid salt.

In addition, the microorganisms may be allowed to coexist with other substances having no adverse effect on the improvement of storage stability and generally used in the field of pharmaceutical, food, or feed.

When the dried microorganisms are allowed to coexist with the L-arginine acidic amino acid salt, they are preferably mixed without being dissolved in a solvent such as an aqueous solvent, e.g., water, an inorganic salt aqueous solution, or a buffer solution; an alcohol, e.g., methanol, ethanol, or glycerol; or a mixture thereof

If the dried microorganisms are allowed to coexist with the L-arginine acidic amino acid salt by mixing, the water content of the resultant mixture does not exceed 5% by weight, and more preferably does not exceed 3% by weight.

The amount of L-arginine acidic amino acid salt is preferably 0.1 part by weight or more, more preferably 1 part by weight or more, still more preferably 10 parts by weight or more, relative to 1 part by weight of the dried microorganisms.

The storage stability of the dried microorganisms can be known as the viability of the microorganisms.

The viability of the microorganisms can be determined as the percentage of the number of viable microorganisms after storage for a predetermined period of time to that before the storage by inoculating the dried microorganisms in a medium capable of growing microorganisms and counting the number of viable microorganisms according to a method such as colony counting before and after the storage.

A composition of the present invention may be the composition containing dried microorganisms and an L-arginine acidic amino acid salt. The composition preferably has a water content of 5% by weight or less, more preferably 3% by weight or less.

The composition of the present invention can be prepared by mixing the dried microorganisms, the L-arginine acidic amino acid salt, and other optional ingredients used in the field of, for example, pharmaceutical, food, or feed.

The composition of the present invention can be used directly for storage of the microorganisms or as a pharmaceutical, a food, a feed, or a starting material thereof.

The composition of the present invention can also be used in combination with preparation bases used in the field of pharmaceutical or food to prepare a preparation, preferably, a solid preparation

Examples of the preparation include tablets, capsules, suppositories, pills, powders, and granules. The tablets can be either enteric-coated tablets or sublingual tablets. The capsules can be enteric-coated capsules.

Examples of the preparation bases used include a excipient, a disintegrant, a binder, and a lubricant.

Examples of the excipient include maltose, trehalose, mannitol, reduced malt sugar syrup, lactitol, xylitol, sorbitol, erythritol, crystalline cellulose, and low-substituted hydroxypropylcellulose

Examples of the disintegrant include carboxymethylcellulose, calcium carboxymethylcellulose, sodium carboxymethylcellulose, crospovidone, sodium croscarmellose, sodium glycolate, and starches such as corn starch, potato starch, and partially pregelatinized starch.

Examples of the binder include polyvinylpyrrolidone, pullulan, methylcellulose, hydroxypropylcellulose, polyvinyl alcohol, gelatin, agar, and pullulan.

Examples of the lubricant include sucrose fatty acid esters; stearic acid; metal stearates such as magnesium stearate, calcium stearate, and sodium stearyl fumarate; glycerol fatty acid esters; and hydrogenated oils and fats.

The ratio of the excipient, the disintegrant, the binder, and the lubricant in the composition of the present invention are not particularly limited as long as it is in the range of amounts generally used for preparations.

In addition to the above preparation bases, the composition of the present invention may contain optional ingredients such as a sweetener, an acidulant, a coloring agent, a flavor, an antioxidant, and a plasticizer.

Examples of the sweetener include saccharin sodium, dipotassium glycyrrhizinate, aspartame, stevia, thaumatin, sucralose, glucose, fructose, and saccharose.

Examples of the acidulant include citric acid, tartaric acid, and malic acid.

Examples of the coloring agent include Food Yellow No. 5, Food Red No. 2, and Food Blue No. 2.

Examples of the flavor include lemon flavor, lemon lime flavor, grapefruit flavor, apple flavor, and orange flavor.

Examples of the antioxidant include tocopherol and cysteine hydrochloride.

Examples of the plasticizer include calcium phosphate, calcium hydrogen phosphate, and fine silicon dioxide powder.

The ratio of the sweetener, the acidulant, the coloring agent, the flavor, the antioxidant, and the plasticizer in the composition of the present invention are not particularly limited as long as it is in the range of amounts generally used for preparations.

The composition of the present invention can also contain ingredients other than above, including a carbohydrate such as dextrin, niacin, vitamins, minerals such as sodium, a desiccant such as fine silicon dioxide powder, and an anticaking agent such as calcium silicate, synthetic aluminum silicate, or talc.

The content of the dried microorganisms in the composition of the present invention is preferably 0.01% to 90% by weight, more preferably 0.1% to 70% by weight, still more preferably 0.1% to 50% by weight.

The content of the L-arginine acidic amino acid salt in the composition of the present invention is preferably 10% to 99.99% by weight, more preferably 30% to 99.9% by weight, still more preferably 50% to 99.9% by weight.

To effectively improve the storage stability of the dried microorganisms, the content of the L-arginine acidic amino acid salt is preferably adjusted to 0.1 parts by weight or more, more preferably 1 part by weight or more, still more preferably 10 parts by weight or more, relative to 1 part by weight of the dried microorganisms.

Process for producing tablets and hard capsules as examples of the composition of the present invention will be described.

For example, a tablet can be produced by a method (hereinafter, referred to as a "direct tableting method") in which dried microorganisms, the L-arginine acidic amino acid salt, the preparation bases, and, if required, the above-described ingredients other than the preparation bases are mixed, and the resultant mixture is compression-molded; a method in which dried microorganisms, the L-arginine acidic amino acid salt, the preparation bases, and, if required, some of the above-described ingredients other than the preparation bases are granulated, the resultant granules are mixed with the remaining ingredients, and the resultant mixture is compression-molded; or a method in which dried microorganisms, the L-arginine acidic amino acid salt, the preparation bases, and, if required, all of the above-described ingredients other than the preparation bases are granulated, and the resultant granules are compression-molded. However, the direct tableting method is preferably used.

The apparatus used for compression molding is not particularly limited and may be, for example, a compression machine such as a rotary compression molding machine or a hydraulic press machine.

Enteric-coated tablets can be produced by coating surfaces of tablets formed by compression molding with a base generally used for enteric coating, such as a shellac solution, a zein solution, or cellulose acetate using, for example, a coating pan. Sublingual tablets, on the other hand, can be produced by tableting at low pressure.

Hard capsules can be produced by mixing and stirring the dried microorganisms, the L-arginine acidic amino acid salt, the preparation bases, and the other optional ingredients and encapsulating the mixture in hard capsules using an encapsulator. If required, the hard capsules are hermetically sealed by a general method. Enteric-coated hard capsules can be produced by coating hard capsules produced in the above manner with a base generally used for coating in production of enteric-coated capsules, such as a shellac solution, a zein solution, or cellulose acetate, by a general method.

The composition of the present invention may be stored by any method capable of storing dried microorganisms. Preferably, the composition is stored by standing in a light-shielded container. More preferably, the composition is sealed and stored in a hermetic container.

The composition of the present invention may be stored at any temperature within a general temperature range, preferably, at 50°C or less.

When the composition of the present invention is administered to a human or nonhuman animal, the dosage thereof depends on, for example, the purpose and form of administration, the age, weight, and symptom of the human or nonhuman animal to be administered, and the type of microorganisms. With dried lactic acid bacteria taken as an example, the number of dried lactic acid bacteria used per dose for each adult human is preferably 1 x 10⁵ to 1 x 10⁹ CFU (colony-forming units), more preferably 1 x 10⁶ to 1 x 10⁹ CFU.

### EXAMPLE 1

L-arginine L-glutamate (manufactured by Kyowa Hakko Kogyo Co., Ltd.; the same product was used throughout the examples) and D-mannitol (D-mannitol (for oral use), manufactured by Nikken Fine Chemical Co., Ltd.; the same product was used throughout the examples) were dried at 70°C for 60 minutes using a constant-temperature dryer.

The dried L-arginine L-glutamate and D-mannitol were dried at 105°C for 240 minutes using a constant-temperature dryer. According to the weight difference before and after the drying, the water contents of the L-arginine L-glutamate and D-mannitol dried at 70°C were determined to be 0.5% by weight and 0.1% by weight, respectively.

Then, 2,400 g of the prepared L-arginine L-glutamate, having a water content of 0.5% by weight; 102 g of a dried product of lactic acid bacteria (FD Bifidus ATK, manufactured by Kyowa Hi Foods Co., Ltd., which contained Bifidobacterium longum in an amount of 3.5 x 10⁹ cells/g or more; the same product was used throughout the examples); 450 g of crystalline cellulose (Avicel FD-101, manufactured by Asahi Kasei Corporation; the same product was used throughout the examples); 30 g of magnesium stearate (manufactured by San-Ei Gen F.F.I., Inc.; the same product was used throughout the examples); and 18 g of tricalcium phosphate (manufactured by Taihei Chemical Industrial Co., Ltd.; the same product was used throughout the examples) were mixed and stirred to prepare mixed powder A.

A mixed powder B was prepared in the same manner as the mixed powder A except that 2,400 g of the prepared D-mannitol, having a water content of 0.1% by weight, was used.

According to the method described in Example 1, the water contents of the mixed powders A and B were determined to be 1.7% by weight and 1.4% by weight, respectively.

The mixed powders A and B are prepared into tablets A and B, respectively, having a diameter of 9 mm and a weight of 300 mg/tablet by tableting using the rotary tableting machine VIRGO 524 (manufactured by Kikusui Seisakusho Ltd.; the same apparatus was used throughout the examples).

According to the method described in Example 1, the water contents of the tablets A and B were determined to be 1.8% by weight and 1.4% by weight, respectively.

The tablets A and B were pulverized, were suspended in sterile water, and were applied to Nissui BL agar medium (manufactured by Nippon Suisan Kaisha, Ltd.) to' grow and count colonies. As a result, the numbers of viable lactic acid bacteria in the tablets A and B were calculated. The number of viable bacteria in the tablets A and B were 8.2 x 10⁸ cells/g and 5.6 x 10⁸ cells/g, respectively.

The tablets A and B were sealed and stored in a hermetic container at 40°C for 14 days.

The numbers of viable bacteria in the tablets after the storage were calculated in the same manner as before the storage.

According to the numbers of viable bacteria in the tablets before and after the storage, the viability of the lactic acid bacteria in the tablets A, which contained L-arginine L-glutamate, was 26.8%, and the viability of the lactic acid bacteria in the tablets B, which contained D-mannitol, was not more than 0.001%.

Thus, the presence of L-arginine L-glutamate in the tablets containing the dried lactic acid bacteria improved the storage stability of the dried lactic acid bacteria.

### EXAMPLE 2

Firstly, 50 g of the L-arginine L-glutamate prepared in Example 1, having a water content of 0.5% by weight; 3.4 g of dried lactic acid bacteria, 43 g of crystalline cellulose, 3 g of a sucrose fatty acid ester (DK Ester F20W, manufactured by Nagase & Co., Ltd.), and 0.6 g of tricalcium phosphate were mixed and stirred to prepare a mixed powder C.

Then, a mixed powder D was prepared according to the method described in Example 1 in the same manner as the mixed powder C except that 50 g of dried L-arginine hydrochloride (manufactured by Kyowa Hakko Kogyo Co., Ltd.) having a water content of 0.3% by weight was used.

According to the method described in Example 1, the water contents of the mixed powders C and D were determined to be 2.6% by weight and 1.8% by weight, respectively.

The numbers of viable lactic acid bacteria in the mixed powders C and D were calculated according to the method described in Example 1, and the numbers of viable bacteria in the mixed powders C and D were 5.4 x 10⁸ cells/g and 6.9 x 10⁸ cells/g, respectively.

The mixed powders C and D were charged into aluminum-deposited bags, were sealed therein with a sealer, and were stored at 40°C for 14 days.

The numbers of viable bacteria in the mixed powders after the storage were calculated in the same manner as before the storage.

The numbers of viable bacteria in the tablets before and after the storage, the viability of the lactic acid bacteria in the mixed powder C, which contained L-arginine L-glutamate, was 24.1%, and the viability of the lactic acid bacteria in the mixed powder D, which contained L-arginine hydrochloride, was not more than 0.01%.

Thus, the coexistence of the dried lactic acid bacteria with L-arginine L-glutamate improved the storage stability of the dried lactic acid bacteria more significantly than the coexistence of the dried lactic acid bacteria with L-arginine hydrochloride.

### Industrial Applicability

The present invention can provide a method of improving storage stability of dried microorganisms, a composition comprising dried microorganisms with improved storage stability and a process for producing the composition, and a method of storing dried microorganisms.

## Claims

1. A method of improving storage stability of dried microorganisms, which comprises allowing the dried microorganisms to coexist with an L-arginine glutamate or L-arginine aspartate.

2. A process for producing a composition comprising dried microorganisms, which comprises the step of mixing the dried microorganisms with an L-arginine glutamate or L-arginine aspartate.

3. The process according to Claim 2, wherein the composition is a preparation.

4. A composition comprising dried microorganisms and an L-arginine glutamate or L-arginine aspartate.

5. A preparation comprising dried microorganisms and an L-arginine glutamate or L-arginine aspartate.

6. A method of storing dried microorganisms, which comprises storing the dried microorganisms in the presence of an L-arginine glutamate or L-arginine aspartate.

## Patentansprüche

1. Verfahren zum Verbessern der Lagerstabilität von getrockneten Mikroorganismen, bei dem man die getrockneten Mikroorganismen mit einem L-Argininglutamat oder L-Argininaspartat koexistieren lässt.

2. Verfahren zur Herstellung einer Zusammensetzung, die getrocknete Mikroorganismen umfasst, welches Verfahren die Stufe des Mischens der getrockneten Mikroorganismen mit einem L-Argininglutamat oder L-Argininaspartat umfasst.

3. Verfahren nach Anspruch 2, wobei die Zusammensetzung eine Zubereitung ist.

4. Zusammensetzung, umfassend getrocknete Mikroorganismen und ein L-Argininglutamat oder L-Argininaspartat.

5. Zubereitung, umfassend getrocknete Mikroorganismen und ein L-Argininglutamat oder L-Argininaspartat.

6. Verfahren zum Lagern von getrockneten Mikroorganismen, bei denen man die getrockneten Mikroorganismen in der Gegenwart eines L-Argininglutamats oder L-Argininaspartats lagert.

## Revendications

1. Procédé d'amélioration de la stabilité au stockage de micro-organismes séchés, qui comprend le fait d'amener les micro-organismes séchés à coexister avec un L-arginine glutamate ou L-arginine aspartate.

2. Procédé pour produire une composition comprenant des micro-organismes séchés, qui comprend l'étape de mélange des micro-organismes séchés avec un L-arginine glutamate ou L-arginine aspartate.

3. Procédé selon la revendication 2, où la composition est une préparation.

4. Composition comprenant des micro-organismes séchés et un L-arginine glutamate ou L-arginine aspartate.

5. Préparation comprenant des micro-organismes séchés et un L-arginine glutamate ou L-arginine aspartate.

6. Procédé de stockage de micro-organismes séchés, qui comprend le stockage des micro-organismes séchés en présence d'un L-arginine glutamate ou L-arginine aspartate.
